(19) Europäisches Patentamt
European Patent Office
Office européen des brevets

(11) **EP 4 198 881 A1**

(12) **EUROPEAN PATENT APPLICATION**

(43) Date of publication:
**21.06.2023 Bulletin 2023/25**

(21) Application number: **21214586.6**

(22) Date of filing: **15.12.2021**

(51) International Patent Classification (IPC):
***G06T 7/00*** (2017.01)

(52) Cooperative Patent Classification (CPC):
**G06T 7/0012;** G06T 2207/10081;
G06T 2207/10116; G06T 2207/30004;
G06T 2207/30016; G06T 2211/408

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **SIMON, Matthias**
  **Eindhoven (NL)**

• **MENSER, Bernd**
  **Eindhoven (NL)**
• **RUETTEN, Walter**
  **Eindhoven (NL)**
• **ENGEL, Klaus Jürgen**
  **Eindhoven (NL)**
• **SCHÄFER, Dirk**
  **Eindhoven (NL)**
• **THRAN, Axel**
  **Eindhoven (NL)**

(74) Representative: **Philips Intellectual Property &
Standards
High Tech Campus 52
5656 AG Eindhoven (NL)**

(54) **IMPROVED WATER MAP CALCULATION IN SPECTRAL X-RAY**

(57) System (S-SYS) and related method for determining a material density map for a target material. The system (S-SYS) may receive spectral data representable in a two-dimensional data space. The spectral data may include measurements acquired by a spectral imaging apparatus of an object in a three-dimensional image domain of the spectral imaging apparatus. The system determines clusters in the data space, one indicative of the target material, the target material cluster, and clusters indicative auxiliary materials, the auxiliary material clusters. The system may determine a mutual geometrical constellation of the clusters. The system determines the material density map based on the geometrical constellation so determined.

**FIG. 7**

EP 4 198 881 A1

**Description**

FIELD OF THE INVENTION

The invention relates to a system and related method for determining a material density map for a target material, to an imaging arrangement, to a computer program element and to a computer readable medium.

BACKGROUND OF THE INVENTION

According to a 2019 fact sheet of the WHO (World Health Organization), available online at https://www.who.int/news-room/fact-sheets/detail/the-top-10-causes-of-death, stroke clocks in second in the top 10 of leading death causes. This amounted to 11% of total deaths worldwide in 2019. The condition of stroke, an obstruction of blood vessels in the brain, may cause, if not death, life changing disabilities. Quick diagnosis and treatment are called for in a suspected case. Edema, an accumulation of water into brain cells have been found to be an indicator for the presence of stroke. 3D Imaging modalities such as X-ray computed tomography (CT) are important diagnostic tools. They allow obtaining 3D imagery of the brain which can be analyzed for edema to support rapid diagnosis. Spectral CT, as opposed to conventional energy integrating systems, allows for energy resolved imaging. The energy dependency of the attention coefficient, the main contrast conferring mechanism in CT or X-ray-based-imaging in general, is harnessed in spectral imaging to obtain a range of useful image types. These include material specific base images, imagery that represent contribution of Compton scattering versus Photoelectric effect, virtual mono-energetic images and others.

Water maps, a representation of spatial distribution of water in the imaged brain, have been computed based on spectral data to support stroke diagnosis. See for example K Noguchi et al in "A Novel Imaging Technique (X-Map) to Identify Acute Ischemic Lesions Using Noncontrast Dual-Energy Computed Tomography", published in J Stroke Cerebrovasc Dis, vol 26(1):34-41, (2017). However, such known approaches require a priori knowledge of precise attention values which may not always be feasible. Use of stock values from reference tables have been found to yield inferior results.

SUMMARY OF THE INVENTION

There may therefore be a need for improved spectral data-based generation of maps for a spatial distribution of a target material in a region interest.

An object of the present invention is achieved by the subject matter of the independent claims where further embodiments are incorporated in the dependent claims. It should be noted that the following described aspect of the invention equally applies to the related method, to the imaging arrangement, to the computer program element and to the computer readable medium.

According to a first aspect of the invention there is provided a system for determining a material density map for a target material, configured to:-

- receive at least spectral data representable in an at least two-dimensional data space, the spectral data including, or based on, measurements acquired by a spectral imaging apparatus of at least a part of an object in a three-dimensional image domain of the spectral imaging apparatus;
- determine clusters in the data space, one indicative of the target material, the target material cluster, and at least one other cluster indicative of at least one auxiliary material, the auxiliary material cluster(s);
- determine a mutual geometrical constellation of the clusters; and
- determine the said material density map based on the geometrical constellation so determined.

In embodiments, the system is configured to visualize said material density map in the said imaging domain on a display device.

In embodiments, the determining by the system of the (mutual) geometrical constellation of the clusters by the system includes determining at least one orientation of one of the clusters relative to the at least one other cluster.

In embodiments, the determining by the system of the said material density map by the system includes dimensionally reducing the said spectral data.

In embodiments, the determining by the system of the said material density map by the system includes projecting the auxiliary material cluster on a subspace defined by the target material cluster.

In embodiments, the said subspace includes a (geometrical) line.

In embodiments, the clusters and/or at least one respective orientation is determined by any one or more of: i) principal component analysis, PCA, ii) a trained machine learning model, iii) segmentation operation. The same algorithm such as PCA may be used for both, clustering and determination of geometrical constellation (such as direction), or separate algorithms can be used for each. Instead of PCA, factor analysis, or projection-based approaches may be used.

In embodiments, the spectral data is de-noised data, denoised by a denoiser of the system or of a denoiser external to the system.

In embodiments, the system includes or is coupled to a user interface configured to allow a user to define the said clusters and/or their orientation based on visual representation of the spectral data on the, or a, display device.

In embodiments, the user interface is a graphical user interface.

In embodiments, the spectral imaging apparatus is any one of: i) an X-ray imager, ii) a computed tomography, CT, scanner iii) or tomosynthesis scanner.

In embodiments, the determining by the system of the orientation of the target material cluster is based on at least one value in the auxiliary material cluster.

In embodiments, the material density map is determined separately for different parts of the image domain.

In embodiments, the at least one auxiliary material includes one or more of grey matter and white matter, and/or wherein the target material includes water or cerebrospinal fluid.

In another aspect there is provided an imaging arrangement comprises a system any one of the above mentioned embodiment, and the spectral imaging apparatus.

In another aspect there is provided a computer-implemented method for determining a material density map for a target material, comprising:-

- receiving at least spectral data representable in an at least two-dimensional data space, the spectral data including, or based on, measurements acquired by a spectral imaging apparatus of at least a part of an object in a three-dimensional image domain of the spectral imaging apparatus;
- determine clusters in the data space, one indicative of the target material, the target material cluster, and at least one other cluster indicative of at least one auxiliary material, the auxiliary material cluster(s);
- determine a mutual geometrical constellation of the clusters; and
- determine the said material density map based on the geometrical constellation so determined.

In another aspect there is provided a computer program element, which, when being executed by at least one processing unit, is adapted to cause the processing unit to perform the method.

In another aspect there is provided at least one computer readable medium having stored thereon the program element, or having stored thereon, the machine learning model.

The proposed system allows computing the map without prior knowledge of predefined attenuation values for the target material and the auxiliary material(s). Instead, the spectral data for a given patient or object is analyzed and the target material density map is computed by using the information in the spectral data collectively in a clustering-based approach. In particular, it is the geometry of the spectral data that is analyzed to compute the map. The geometric constellation of the clusters are processed in computing the target material density map. Computing water map based on spectral data in brain imaging to support stroke diagnosis is an application envisaged herein in preferred embodiments, but the principles disclosed herein may be used instead in other medical or even non-medical imaging tasks in relation to other target materials, as required. There is no reliance on explicit predefined material specific attenuation values from outside sources. With the proposed system, "bespoke" attenuation values as applicable to the concrete patient and imaging situation at hand are accounted for collectively and implicitly, based on the concrete, specific, given spectral data themselves to so compute the density map. Highly accurate results with low or no artifacts are obtainable herein. The proposed system harnesses a property of directionality of the clusters discovered herein which ensures the orientation per cluster is well defined. It appears this directionality property stem from a particular noise behavior of the spectral data. The proposed method produces the target material density map with contrast modulated by a deviation (excess or deficit) of the target material from a normal or expected density/concentration, instead of representing absolute density/concentration. Thus, the proposed method and system is in particular suitable for applications, such as in edema detection, where there already is a residual, normal component of the target material present in the one or more auxiliary materials. Contrast in the target material is thus focused on said deviation.

In more detail, and with reference to brain imaging as an example, the mean Hounsfield (HU) values of grey matter ("GM") and white matter ("WM") tissue is not required herein to calculate a water map for example. Specifically, and in embodiments, the HU values for geometric cluster constellation in terms of projection direction and base line can be computed without any prior knowledge of such HU values for the materials involved. Prior knowledge of such material specific HU values applicable to a concrete patient case for clinical imagery proved remarkable difficult. Reasons for this include:

Upfront calculation of the HU values from general assumptions of the elemental tissue composition does not work well since the elemental composition and density might differ from patient to patient as well as within one patient's image. The HU scale of the image needs to be calibrated absolutely, which cannot be guaranteed in most cases. Derivation of the GM and WM values directly from clinical brain images is challenging, too, due to several reasons:

intra-patient variation due to remaining beam hardening in case of image based material separation
variation across the brain volume due to scattered X-ray and insufficient scatter correction
a clear segmentation between GM and WM is difficult due to partial voxel effects and noise, which leads to strongly overlapping distributions of the HU values for GM and WM.

What is proposed herein in embodiments is a water map calculation based on preferably projection-based material separation, and a 2D density plot of attenuation values (referred to herein as the "A-space") for two different virtual mono-energetic images, e.g. at 40 keV and 70 keV after bone-water or photo-Compton material decomposition. An optional denoising step is included in the processing after material decomposition, taking into account anti-correlated nature of the noise in the base images. The attenuation values of voxels containing GM and WM appear in a distribution in the 2D plane of the A-space. The width of this distribution is mainly determined by a certain amount of noise in the basis images. The voxel distribution of each material has an ellipsoidal form with a long (major) and short (minor) axis. A projection line, along which the voxels are projected onto the GM-water line, is determined from the orientation of the long axis of the ellipsoidal voxel distribution of GM and WM. This axis is oriented in the A-space with the same slope as the connecting line between the centers of the GM and WM HU distribution and can be much easier derived than the center points of each material's HU distribution. This is especially the case if both voxel HU value distributions of GM and WM overlap. The target line between water or cerebrospinal fluid (CSF) and GM or WM can also be determined by an automated step from the image values.

The A-space is at least 2-dimensional ("2D"). A-space, also referred to as attenuation space, is different from spatial domain. The A-space may be understood as a transformed representation of the spectral data with dimensions spanned by different manners of attenuative interaction of X-ray and matter. For example, such manners of different interaction may include attenuation at different energies as brought about by the imaging apparatus using detector or source side hardware solutions. Other examples include attenuation due to Compton scattering versus photoelectric absorption ("photo"), or attenuation caused by different base materials (bone and water), etc. The A-space may be of dimension N higher than two, for example for an $N$-layer system for definition of N base functions. For $N \geq 2$, projection in A-space may be used to reduce the dimensionality of the parameter space/A-space to a smaller dimension such as $N\text{-}k, \ k < N$. For example, for the water map case and $N= 2$ and $k=1$, the data points are reduced to a subspace, the water base line.

The target material density map computed herein is a quantitative representation of the spatial distribution in spatial domain of the target material density in a mix of the one or more auxiliary materials (and possibly additional auxiliary materials). The density may be converted into a concentration. For example, the map may represent the relative content of the target material in the material mix in which target material is embedded. The density may be represented in the map by attenuation values such as in the Hounsfield unit scale or in any other suitable quantity. The target material map (eg, water map in brain imaging) may be unitless. The target material map is preferably normalized. For example, in a predefined interval, such as the unit interval, "0" may represent "normal water density of healthy grey mater" and "1" may represent "water density in cerebrospinal fluid".

The spectral imaging apparatus may include any type of imaging equipment with X-ray based acquisition and configured for spectral imaging, such as by source-side or detector side configurations. Diagnostic or therapeutic X-ray imagers are envisaged herein, and so are mobile or fixed installations.

The system may operate fully automatically or based on user input. Thus, the user may by way of a GUI or other specify direction/orientation and optionally position of clusters.

In some embodiments, the orientation/direction is determined based on barycenter or other designated point for each cluster. Lines are then run through the points to define base line for target cluster and projection direction though auxiliary clusters, respectively.

"Spectral data" refers to data derived from raw data of the spectral imaging apparatus, representing aspects of matter-versus-X-ray interaction. For example, the aspects may include attenuation values for different manners of matter-versus-X-ray interaction, represented so as to relate attenuation values for one manner of matter-versus-X-radiation interaction to attenuation values for at least one other manner of matter-versus-X-radiation interaction. For example, in a two-dimensional data space, such as A-space, the attenuation values may relate to interactions including Compton scattering (scatter coefficient) versus photelectric absorption (absorption coefficient). In another example, the two-dimensional data space might be extended to a three-dimensional case which additionally include the X-ray interaction around the K-edge of a particular chemical element like e.g. Iodine or Gadolinium. Alternatively to such "manners of X-ray interaction", "aspects of matter-versus-X-ray interaction" may include the energy dependency of materials. For example, "spectral data" may include the X-ray attenuation for different X-ray energies, the spectral data thus including attention values from two mono-energetic images, represented with one set of attenuation values at one energy versus another set at least one other energy. Alternatively, the attenuation values relate to interaction with two or more different basis materials, sufficiently different in their atomic number, the spectral data based on a material decomposition/separation operation. For example, these basis materials might be formed by water, bone, one or more contrast agents, or different combinations thereof. Still other aspects of interaction are not excluded herein, such as effective attenuation values obtained from two different effective X-ray spectra.

"*cluster(s)* " is/are a subset of preferably A-space. The cluster as determined herein have direction (are elongated, such as ellipses or ellipsoid or other structures) and allow for definition of such as direction, such as major axis of the ellipse/ellipsoid.

*"density"* of the material (target or auxiliary) as used herein relates to relative density of the material in the mixture with the other one or more materials. Relative density allows describing accumulation (or depletion) phenomena where more material of one type accumulates in a given volume of the mixture, thus causing relative density changes, such as for example with water accumulation in edema. Relative density may be also described in terms of concentration.

*"User"* relates to a person, such as medical personnel or other, operating the imaging apparatus or overseeing the imaging procedure. In other words, the user is in general not the patient.

*"Object"* is used herein in the general sense to include animate "objects" such as a human or animal patient, or anatomic parts thereof but also includes inanimate objects such as an item of baggage in security checks or a product in non-destructive testing. However, the proposed system will be discussed herein with main reference to the medical field, so we will be referring to the

"object" as "the patient" or a part of the patient, such as an anatomy or organ, or group of anatomies or organs of the patient.

BRIEF DESCRIPTION OF THE DRAWINGS

Exemplary embodiments of the invention will now be described with reference to the following drawings, which, unless stated otherwise, are not to scale, wherein:

Figure 1 is a schematic block diagram of a spectral imaging system;
Figure 2 is an illustration of points in an attenuation data space;
Figure 3 illustrates a clustering operation in attenuation data space according to one embodiment;
Figure 4 shows a schematic block diagram of a quantitative spectral data processing system configured for computing a density map of a target material;
Figure 5 illustrates an operation of determining a geometric constellation of clusters in attenuation data space according to one embodiment;
Figure 6 illustrates a clustering operation in attenuation data space facilitated by user input;
Figure 7 illustrates an operation of determining a geometric constellation of clusters in attenuation data space according to another embodiment; and
Figure 8 shows a computer-implemented method of generating a density map of a target material based on spectral data.

DETAILED DESCRIPTION OF EMBODIMENTS

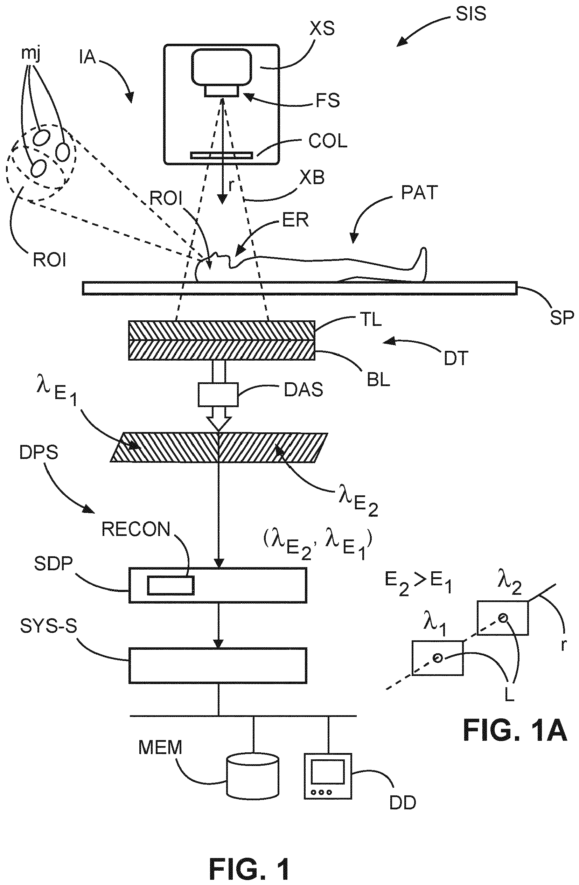

Figure 1 is a schematic block diagram of a spectral imaging arrangement SIS. Broadly, the system includes the imaging apparatus (briefly referred to herein as "imager") IA having a detector system DT, and a data processing system DPS configured to process data generated by the detector system DT during data acquisition.

The data processing system DPS includes a system S-SYS for generating a density map of a target material in an imaged region of interest ("ROI"). The system may be used in brain imaging for quick and accurate identification of water accumulation in tissues (edema) in a suspected stroke patient. Before explaining the novel map generator system S-SYS in more detail, reference is first made to components of the imaging equipment IA to facilitate exposition.

The imaging apparatus IA may be a CT scanner. The scanner IA may include a stationary gantry and a rotating gantry (not shown). The rotating gantry is rotatably supported by the stationary gantry and rotates around an examination region, and a portion of an object or subject therein about a Z-axis. A radiation source XS, such as an X-ray tube, is supported by and rotates with the rotating gantry around the examination region ER. The radiation source XS emits in general wideband polychromatic X-ray radiation XB that may be optionally collimated to form generally a fan, wedge, or cone shaped X-ray radiation beam that traverses the examination region ER.

A radiation sensitive detector array of pixels of a detector system DT may subtend an angular arc opposite the radiation source XS across the examination region. The detector array includes one or more rows of the detector pixels that are arranged with respect to each other along the Z-axis and operate to detect X-ray radiation traversing the examination region ER and hence parts of the patient PAT. The detector pixels generate the projection (raw) data.

A subject support SP, such as a couch, supports a subject PAT (e.g., a patient) in the examination region. The subject support SP is movable in coordination with performing an imaging procedure. This allows moving the subject PAT or object with respect to the examination region for loading, scanning, and/or unloading the subject or object.

An operator console (not shown) may include a human readable output device such as a display monitor DD, etc. and a user input device such as a keyboard, mouse, etc. The console OC further includes at least one processor (e.g., a central processing unit (CPU), a microprocessor, GPU/TPU etc.) and computer readable storage medium (which excludes transitory medium) such as physical memory. The operator console allows user to control the imaging procedure.

Whilst the principles disclosed herein are described with main reference to CT or other volumetric/rotational imaging modalities such as C-arm imagers or similar, they are of equal application to projection imaging in radiography.

During imaging/acquisition operation, the patient PAT, or at least a region of interest ("ROI") of patient, resides in the examination region. For example, the patient may lie on the patient couch SP arranged at least partly inside a donut shaped CT examination region.

The X-ray source XS is energized. X-rays XB emerge from the source XS, traverse the examination region ER and the ROI/patient PAT, and are then registered at the far end at the X-ray sensitive pixels of the X-ray detector DT.

The impinging X-ray XB causes the X-ray sensitive pixels to respond with electrical signals. The electrical signals are processed by data acquisition circuitry DAS of the scanner IA to produce digital projection raw data.

Spectral imaging capability may be implemented by detector-side or source-side solution. A detector-side solution for dual energy imaging is shown in Figure 1. The detector DT comprises two layers, a (relative to the X-ray source XS placed atop) top layer TL and a bottom layer BL of X-ray sensors, in order such that the X-ray beam trajectory first passes the top layer, then the bottom layer. This "dual energy" arrangement allows detecting high energy E2 projection data at the bottom layer BL and lower energy projection data E1 at the top layer TL. More than two such layers (multi-layer detector) can be used if resolution into more than two energy windows is required. In embodiments, resolving into two or more than two energy levels may be obtained at the detector-side by having a specially configured detector DT that is equipped with counting circuitry (not shown). The counting circuitry classifies incoming intensities in projection domain into different energy bins against a set of energy thresholds. Source-side solution are also envisaged, such as dual-or multi-source imagers, or those with a single source XS equipped with kVp switching circuitry, or those applying a varying X-ray filtration as to generate different X-ray spectra. In further embodiments, spectral imaging capability may be implemented by repeated imaging procedures of a system used with different setup parameters as to obtain a volumetric scan for different spectra. For example, a common X-ray imaging system could perform the same imaging procedure with two or more different X-ray tube voltage settings generating at least two-dimensional spectral data. In further embodiments, a multitude of simultaneous or non-simultaneous X-ray beams may be used, each having a different spectral setup. For example, two or more X-ray sources with corresponding two or more detectors forming separate beams in space may be applied to acquire spectral data.

X-ray spectral imaging is based on X-ray spectral data, or spectral data S for short. Spectral data S is, or relates to, multi-dimensional detector data as indicated in inset Figure 1A. For each direction $r$ of acquisition rotation, and each image location L on the X-ray sensitive layer of the detector DT, there are detected by detector pixels two (or more) different projection data values, indicated schematically as $\lambda 1$, $\lambda 2$.

As an example, dual energy imaging is considered herein where there are exactly two such values, corresponding to different energy levels or intervals/windows E1, E2. The energy levels correspond to different acquisition energies, implementable by detector-side or source-side solutions, each envisaged herein in different embodiments. There may be more than two such levels such as three or more, for example when photon counting detector circuitry is used, also envisaged herein in embodiments. In spectral imaging, such multi-dimensional projection data is processed. More generally still, spectral imaging is reliant on multiple aspects of matter versus X-ray interaction. One such aspect of interaction is in terms of said different energy levels of photons in the imaging beam XB. Other aspects of interaction that give rise to such multi-dimensional spectral data, and as envisaged herein, includes multiple interaction mechanisms, such as absorption by photo-electric effect versus Compton scattering for example. Other interaction effects may be considered instead or in addition. Yet other such aspects of interaction that give rise to spectral data is the energy dependent interaction with two (or more) material types (sometimes referred to as basis materials).

In addition to being configured for spectral imaging, the imager IA is also configured herein in preferred embodiments for 3D imaging, involving two conceptual data spaces, projection domain and (spectral) image domain. Projection domain relates to projection data representing line integrals and collected at detector's X-ray sensitive detector surface formed from pixels. The detector pixel layout is preferably 2D, such as a plane or a curved surface as required, depending on the detector and acquisition geometry design. For example, cone beam CT (CBCT) is envisaged herein in some embodiments or other "fully 3D" tomographic setups. Helical CT is also envisaged herein, but slice-wise scanning protocols are not excluded herein. Projection domain represents the projection data acquired by the imager's detector DT at different projection directions as realized during rotation of the source XS around the ROI. Fourth generation type scanners are also envisaged where there are multiple sources around the ROI so no mechanical rotation is required. A reconstructor RECON is used to process projection data into cross sectional (tomographic) images in an image volume.

Image domain is a portion of 3D space in the examination region ER where the ROI is located. Imaging domain is conceptually made of a 3D grid of image elements or voxels. The reconstructor implements a computational spectral tomographic reconstruction algorithm to compute the image volume in the image domain. Computing the image volume results in populating the voxels with image values. The region of interest represents an organ or group of organs considered for the current imaging task. Brain imaging is one type of imaging task considered herein in embodiments.

The imager is thus configured not only for spectral but preferably also for 3D imaging. Spectral data includes data in projection domain and/or data in image domain reconstructable from projection data. Amongst other types of spectral data (on which more further below), such spectral data in image domain is also referred to herein as spectral imagery. Spectral imagery may be visualized by visualizer circuitry as a graphics display on a display device DD. The said visualization may include a 3D volume rendering or visualization a slice of such a 3D volume or other portion thereof.

Spectral data processor SDP processes spectral projection data in projection domain and/or spectral image data in the image domain to produce other types of spectral projection data or spectral image data by using any one of a number of different spectral data processing algorithms. Such types of spectral data include virtual mono-energetic images, material images obtained by material decomposition techniques, interaction densities of Compton scattering and photoelectric effect images, etc.

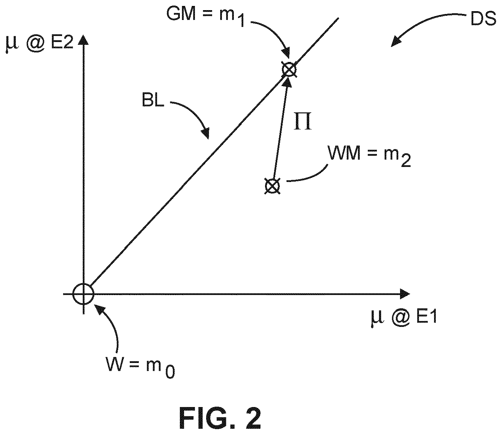

The imaging arrangement includes a novel system S-SYS for quantitative spectral imaging that allows computing, based on any one of the above-mentioned spectral data, density/concentration map for a target material. Before turning to operation of the quantitative spectral data processing system S-SYS in more detail, reference is first made to Figure 2 in order to elucidate concepts of spectral data that may be relevant for the processing envisaged herein.

The above-mentioned spectral data of any type can be formulated in terms of attenuation values. Attenuation values relate to the attenuation experienced by X-radiation as it passes through and interacts with material of which the relevant body portion (including the region of interest) is made up. Diagnostic imaging is mainly envisaged herein. Some types of diagnostic imaging may query a region of interest for the presence of a certain material. A type of diagnostic imaging envisaged herein in embodiments where a need for such querying may arise is in brain imaging. Brain imaging may be called for if patient needs to be examined for strokes. In strokes it has been observed that water may accumulate in brain cells causing a swelling (edema). There are two types of brain cells which manifest in different materials, referred to as grey matter ("GM") and white matter ("WM"). For improved stroke diagnosis, an accurate water map may be beneficial to quantify presence of water in the brain. The water map, or indeed any other material-of-interest map, is a scalar field situated in the 3D examination region where the brain is located. The scalar field assigns to each voxel a respective imageable value. The value represents the amount, or is relatable to such an amount, of water at the respective voxel image domain. The water map may support a user, such as a diagnostician, in correct and rapid stroke diagnose. Suitable therapeutic measures may be put into place quicker, to the benefit of patients thanks to the proposed system S-SYS. Whilst brain imaging is a main application envisaged herein, the proposed set-up for spectral imaging in relation to attenuation values can be used for computing density or concentration maps for any material of interest, whether in brain imaging or when imaging for other organs or tissues. In fact, applications outside the medical field such non-destructive material testing, scanning of luggage at security checkpoints at (air)ports, etc, are not excluded herein, although in the following main reference will be made to brain imaging and water map generation as an example.

In diagnostic tasks as outlined above, the region of interest is assumed to be made up of two or more materials, including the material of interest. The material of interest may be embedded in a mixture or assemblage of one or more additional materials "not-of-interest", referred to herein as auxiliary materials. In the present case of brain imaging, the region of interest may be made up of water (the material of interest) with auxiliary material(s) including GM, WM. The water in turn may be normal water or cerebral fluid or additional fluid causing edema, the presentation of which may be considered an indication for the presence of a medical condition such as stroke or others.

Spectral data S expressed in terms of attenuation values is representable in a multi-dimensional data space DS, also referred herein as the attenuation data space ("A- space"). A-space relates attenuation values of different aspects of X-ray interaction. For example, co-ordinates or dimensions in this at least 2D dimensional data space DS may be made up of attenuation values at different energies as indicated in the example A-space plot representation in Figure 2.

Spectral data when represented in A-space may be considered transformed spectral data, transformed from spatial domain. The spectral data in spatial domain is provided by the spectral data processor system SDS. In spatial domain, spectral data is represented in terms of pixels or voxels spatially. Instead, in A-space, a location represents the number, or relative frequency, of data points in spatial domain that experienced the respective attenuation interaction as indicated by the A-space location. For example, a location in A-space is indicated in Figure 2. The location refers to all voxels where a specific combination of attenuation values at different energies was observed. Each point in A-space may be represented in terms of coordinates ($HU1, HU2$) of Hounsfield units ("HU"). For the case of dual energy imaging, and according to one embodiment of A-space, coordinate $HU1$ is the attenuation in HU at energy E1, whilst coordinate $HU2$ is the attenuation in HU at energy E2. Using Hounsfield units HU does not exclude other possible forms of unit representations, as for example mass densities or other still.

Because attenuation is in general dependent not only on energy but also on the material type encountered by the X-ray photons during interaction, regions of a given pure material in the spatial domain may be represented as a single point in A-space. Thus, in the case for example where the region of interest ROI can be assumed to be made up of three materials (schematically shown to the left in Figure 1), a target material m0 and two or more auxiliary materials m1,m2 (two are shown in Figure 2), such a region can be represented in this data space as 3 data points m0, m1 and m2, defining a certain geometric constellation such as triangle. In a slight abuse of language but in the interest of brevity, we will use $m_j$ to refer to both, the material type and the data point representing this material type in A-space as shown in Figure 2. For example, in brain imaging, one data point represents target material m0 water, whereas the other data points m1 and m2 represent white matter ("WM") and grey matter ("GM") respectively. X-ray attenuation difference between GM and WM is mainly given by the lipid content of WM. Distances between points in A-space represents contrast. It may be noticed that the water point *m0* in the data space DS is located at the origin of the A-space co-ordinate system. However, this is immaterial and is merely a result of the fact that Hounsfield units are used for this representation of the data space as an example. Any other representation may be used instead where the target may not come to lie at the origin of A-space shown in Figure 2. In fact, instead of using the water as the target material, the inter-cerebral fluid ICF may be used, which is in essence water mixed with traces of minerals. Due to the minerals, the ICF material point is not at the origin of the data space co-ordinate system, even if Hounsfield values are used.

The spectral data in spatial domain can be transformed by a suitable transformer T into A-space. Transformer T implements transformation T. The transformer T may be implemented as follows: for each HU combination of suitably small intervals (*ΔHU1,ΔHU2*), the spatial domain spectral data, such as a pair of monoenergetic images $f_{E1}$, $f_{E2}$, is searched. Each voxel in the two images $f_{E1}$, $f_{E2}$ whose value falls into the intervals for the current HU pair is flagged and a counter is set at coordinate (*HU1, HU2*). If the spatial coordinates are tracked and retained in lists as preferred herein, the transformation T from spatial domain into A-space as implemented by transformer T is reversable. A point from A-space may thus be transformed back into points in spatial domain. A-space may be implemented as an associative array, with each entry representing a HU combination and each entry associated with a list of coordinates in spatial domain which data values correspond to the HU combination.

Operation in A-space is beneficial for the proposed qualitative spectral system SYSS for target material density map generation as considered herein. Processing of spectral data in this space allows computing precise material density maps such as water maps with reduced or no artifacts. What is proposed herein is to represent spectral data in this data space and to artificially reduce by the processing of system S-SYS the contrast between the auxiliary materials at the benefit of an increased contrast of the (single) target material of interest *m0,* relative to the auxiliary materials *m1, m2.*

The proposed processing for increasing contrast of the target material at the detriment of contrast amongst the auxiliary materials can be conceptualized as an exercise in dimensional reduction of the said geometrical constellation of the data points *m0,m1,m2* in this data space. Given one of the auxiliary materials, such as grey matter GM=*m1* 1, a mix of this auxiliary material and the target material may be conceptualized as a base line BL, that is a linear/affine space, in this case a geometric line, extending between the target material point m0 and the auxiliary material point GM= *m1.* The other material point WM=m2 may be used instead. The base line in brain imaging may be understood as a "water slope".

Each point on the base line can be represented by a scalar that measures the purity of the mixture between target and one of the auxiliary materials, such as GM at point m1. For example, a point on base line located at m0 would represent a case of pure water. As one moves along the base line, this will represent mixtures of less and less concentration of water with increased concentration of the first auxiliary material m1, such as grey matter, as shown in the Figure 2. Thus, a location on the base line at m1 will thus represent a concentration of pure grey matter and water. It should be understood however that this principle does not necessarily imply a zero concentration of an auxiliary material in point m0, neither a zero concentration of base material in the auxiliary materials at m1 and m2, but respectively in concentrations which serve as reference values. In the example of Figure 2, however, m0 may indicate a substantially zero concentration of WM and GM, while m1 and m2 may indicate pure WM and GM, respectively, which, however, even in healthy state may still contain certain concentrations of water.

The general rationale of the operation of the system S-SYS is now as follows. Each or some voxels x of the ROI in spectral spatial domain may be transformed to points *T(x)* in data space DS, A-space. The transformed points may either lie on this base line BL or not. If the transformed point T(x) does come to lie on the base line BL, then the transformed point's distance (the coordinate in the linear space) on base line BL between the target material and the auxiliary material represents, or can be mapped to, the relative concentration or density of target material at voxel x. If, on the other hand, the transformed point T(x) in spatial domain does not come to lie on the base line, which is likely, this point T(x) may still be projected onto the base line, for instance along a direction parallel to the line through m1 and m2. The location of T(x) after the projection on the base line is then resolved as explained above in terms of a density or concentration value for voxel x. Thus, by this dimensional reduction, implemented as projection operation, contrast between the auxiliary materials GM and WM is reduced, but their contrast relative to the target material is increased, thus yielding high contrast and accurate density maps for the target material.

Thus, "out of base line points" are projected onto the base line to obtain a quantification of the density or concentration of the target material. The projection direction is designated in Figure 2 by Π. The correct projection direction is a function of the auxiliary materials *m1, m2.* In the presence of two materials, as shown in Figure 2, this projection direction Π is preferably given by second line, the projection line, that passes through the two auxiliary material data points *m1,m2* for, in the brain imaging embodiment, white matter and grey matter, respectively.

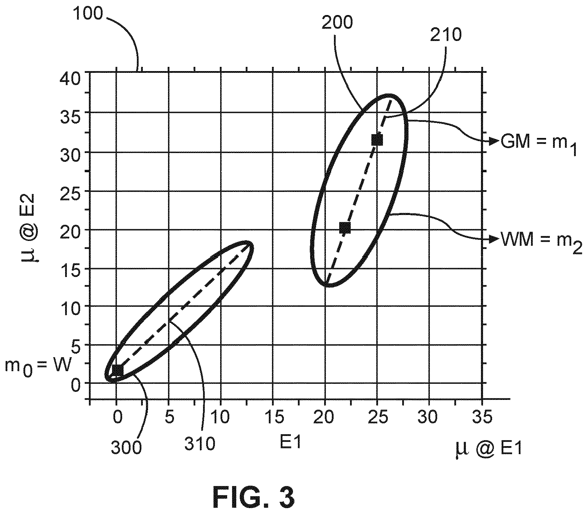

It may appear from the above-described geometry in A-space that in order to define the projection line Π and base line BL, the exact location of the data points *m0,m1,m2* in this data space must be known a priori. For example, assuming attenuation values in terms of Hounsfield units as shown in Figure 1, it may appear as if the above-described dimensional reduction operation may rely on a precise a priori knowledge of the pair of Hounsfield values for each of the three materials involved. But with the proposed system S-SYS configured for such as dimensional reduction operation to compute density map for target material, there is no such reliance on prior knowledge on the attenuation value pairs for the ROI materials, the target material and the auxiliary material(s). In particular, the proposed system S-SYS is capable of generating the water map, without prior knowledge of the attenuation values at different energies for water, GM and WM. With the proposed system S-SYS, there is no need for such prior knowledge and there is no need to estimate those locations in data space as will be explained in more detail in connection with Figure 3 to which reference is now made.

Figure 3 is an illustration of operation of the proposed system according to one embodiment obviating the need for prior-knowledge of coordinates of the three (or only two) material points *m0, m1 and m2* for the generation of target material density map $\delta_{m0}$. Instead of reliance on material point coordinates, system S-SYS is operable in A-space, shown as data space DS,100, to perform a clustering of the collection of transformed points T(x). Initially, when the spectral data is transformed, the transformed points have been found to form a point cloud, rather than three distinct points as idealized in Figure 2, which is due to noise, density fluctuations and other uncontrollable factors. It has been found that the point cloud may be separated into a number of point clusters, the number corresponding to the number of materials under consideration, that is, the target material and the auxiliary material(s). These material clusters *cj,* as they may be aptly called for their correspondence with the ROI materials *mj,* can be analyzed for their mutual geometrical constellation. For example, principle component analysis ("PCA") may be used to define the number of clusters, for example three such clusters, for water, GM and WM. It has been found that because of a specific noise behavior of the spectral data (to be described more fully below), the clusters admit to a definition of directionality. The direction or orientation of each cluster may be used, as has been found, as sufficiently accurate estimates for the above-described projection direction Π and the base line BL. Thus, instead of relying on theoretical, tabled, a priori attenuation values for individual material types, the proposed system is configured to, in embodiments, automatically estimate the projection direction Π and the base line BL direction from the point clouds collectively as illustrated in Figure 3. Semi-automatic embodiments that accept some form of user input are also envisaged herein in alternative embodiments and will be described in more detail below in connection with

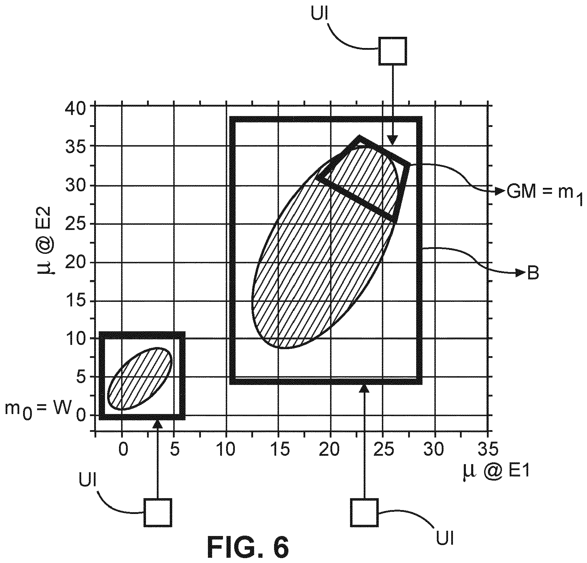

Figure 6. Once the directions Π and BL are known, the points in A-space may be projected on the base line as described above, with subsequent reverse transformation $T^{-1}$ into spatial domain to find the water map or other target material density map.

As said, PCA or least square methods or any other numerical techniques may be in harnessed herein to analyze the point cloud to identify the required number of clusters and compute the projection direction and base line direction. The projection and base line directions may be found as the respective major axis of the elliptic definitions as revealed in the PCA for each point cluster *cj* into which the initial point cloud is separated. PCA as one example envisaged herein for facilitating the dimensional reduction may be done for each of the three materials m0, m1, m2, yielding three directions, one more than needed. Two of these may be averaged or otherwise combined to obtain the two directions BL and Π. Alternatively, GM and WM points may be lumped into a single combined "brain matter" cluster, with one major axis as per PCA to find the projection direction Π. Thus, the brain matter points form a single combined cluster, definable as a single ellipse and thus a single major axis for the combined mixture of brain matter *"m1 + m2"*. The major axis of the single auxiliary material for the considered conglomerate of the two auxiliary materials m1, m2 may be used as the projection direction Π to project onto the base line BL. In Figure 3, baseline BL is indicated as major axis 310 of target material (eg, water) cluster 300. Figure 3 further illustrates major axis 210 of the conglomerate cluster 200 for the two auxiliary materials *m1,m2,* such as GW and WM.

Thus, the relevant directions, the projection direction and base line can be computed fully automatically as directions 310 and 210, respectively in the example illustrated in Figure 3. The density map for target material is then computed as described before by projection onto baseline 310 along direction 210 for all or some points in the point cloud of A-space, and then back-transformation of the respective coordinates along 310 into spatial domain.

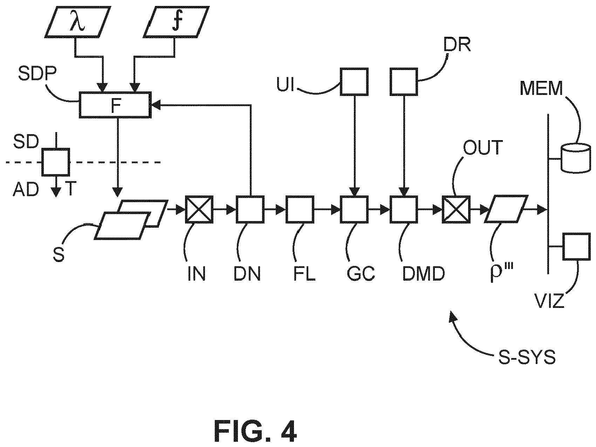

Whilst Figure 3 is an illustration of the conceptional operation of the proposed system S-SYS, such Figure 3 or similar may be rendered graphically on a graphics display visualized by visualizer VIZ, preferably in real-time and dynamically, any one or more of: the point cloud, the clusters found and/or their respective axis. The graphics display may be part of a graphical user interface ("GUI") which may be configured for interactive operation with the user. Similarly in relation to Figures 4-7, which are also envisaged herein for preferably real-time/dynamic visualization as a graphics display for display on a display device DD to yet better support user in using system S-SYS.

Thanks to the clustering operation of the proposed system S-SYS, no (exact) prior knowledge on point coordinates m0, m1, m2 is required. In case of the water map, because water has less attenuation than GW or MW, and is present in pure form of cerebrospinal fluid in large regions of the brain, the cluster more proximal to the origin can be easily identified automatically as the water cluster, thus leaving the other cluster(s) as the GW, WM or GW+WM combined auxiliary material cluster. Thus, for assigning material type to each cluster, a mere comparative knowledge of the attenuating properties of the ROI material is sufficient. This cluster vs material type assignment can be done by a comparative logic of the system S-SYS. Alternatively, user interface UI may be provided, in particular in combination with the above-described visualizations, configured to allow a user to tag or otherwise indicate which cluster relates to the target material, the remaining clusters then being automatically treated as auxiliary materials. Alternatively, additional user input is provided to indicate the material type, such as GM and WM, each of the remaining clusters. Alternatively, all cluster other than the target cluster are actively indicated, the leftover cluster being automatically indicated as the target cluster. The indication functionality by UI may be implemented by a pointer tool (mouse, stylus, etc), by touch screen action, or by keyboard input, as required. For example, the user can touch or click on the visualized cluster and assign a token *"TARGET"* or *"AUX1", "AUX2",* etc.

PCA, whilst mainly envisaged, is not the only algorithm considered herein. Other algorithms for finding clusters and their orientations to define baseline BL and projection direction $\Pi$ include machine learning, or other techniques, least square line or subspace fitting, statistical techniques, and others.

Reference is now made to Figure 4 which shows a schematic block diagram of the system S-SYS for quantitative spectral imaging. In particular, the system S-SYS as explained above is configured for determining the density or concentration map in 3D (in the examination/imaging region) of a target material. For brain imaging, such target material may include water or cerebral spinal fluid CSF. The system operates on spectral data S. Spectral data S may be multi-dimensional spectral projection data $\lambda$ or spectral reconstructions $f$ thereof. Spectral data S is generally representable in spatial domain in terms of voxel or pixel positions in image domain or projection domain. The spatial domain represents locations of interaction with photons of the imaging beam. Spectral data S may be further generated by processing the spectral projection data $\lambda$ or the spectral reconstrued data $f$ by one of the above mentioned spectral data processing algorithms, symbolically represented as a function $F(\lambda), F(f)$, to produce a mono-energetic imagery, material decomposition, Compton scatter vs photoelectric absorption bases image decomposition, etc as explained above. Preferably, as envisaged herein the spectral data S is transformed by transformer T into attenuation data domain. Attenuation data domain may be represented as attenuation data space, A-space, as mentioned earlier. Spectral data S represented in A-space is received at input port IN. The spectral data S in A-space may present as plural data points in a point cloud. An optional denoiser DN is used to denoise the data. Alternatively, the denoising is done in spatial domain before or after applying the spectral data processing algorithm F.

A geometry component GC, such as a clusterer, determines clusters $c_j$ in A-space. The clusters $c_j$ are representative of the target material and at least one, for example two, auxiliary materials. In preferred embodiments, three such clusters are determined, one for brain matter, such as grey matter and/or white matter, and one for the target material, which is CSF or water. Single auxiliary material cluster may be sufficient in some embodiments. The clusters so determined are assumed to span or define a geometric constellation in A-space AD. Attenuation space and cluster constellation is at least 2D but can be 3D or higher dimensional still. There may be provided a filter FL component that filters out subset(s) of points in A-space and the clustering operation is based only on the so filtered subset of points. A thresholding may be used or segmentation based on attenuation values in one or both (or more) energy windows E1, E2. Anatomical knowledge may be taken into account in the filtering. The filtering allows guiding the clustering operation as only points deemed relevant enter into the clustering computation. In addition, a computational burden may be reduced. For example, voxels representing brain ventricles may be excluded, as the ventricles are known to include water-like matter (the said CSF) anyway even for non-pathological cases, and can be left out for the water map to be computed as it is pathological/unnatural accumulation (edema) of water/CSF that is of interest. In general, the filter FL may be configured to exclude voxels of anatomies that are known a prior to include the target material or that are otherwise of no interest for the map to be computed.

Definition of the clusters by geometry component GC may be fully automatic. Alternatively, the cluster definition may be based on or at least a coarse user-based segmentation, or on other user input received through user interface UI, such as a graphical user interface where the clusters in their geometric constellation are visualized by visualizer VIZ on the display device DD. The UI or another UI may be used to tag or otherwise indicate the clusters to define which cluster represents the target and auxiliary material(s), respectively.

The geometry component GC may be operable to determine a respective direction of such clusters. Specifically, the geometry component GC may determine the geometric constellation of the clusters by determining orientations for the clusters, one orientation per cluster. The orientation of the target material cluster is determined as base line BL, whilst an orientation of the auxiliary material cluster(s) are determined as the projection direction $\Pi$. There may be a respective orientation for each auxiliary material cluster which may be combined (eg, averaged) by geometry component into a single orientation $\Pi$. Alternatively, clusters are combined for different auxiliary materials, and a single orientation $\Pi$ is then determined for the combined cluster. One line for the brain matter cluster which may include grey and white matter defines projection direction whilst the base line is determined by the point cloud that represents the target material such as the water. The geometry component GC may use PCA to find clusters, with their orientations given by the respective major axis.

As an alternative, respective barycenter of clusters are computed to obtain three material points (similar to Figure 2), one for the target material cluster, eg for water or CSF, two barycenter points for GM and WM cluster, respectively. The base line may then be defined by the line between the water barycenter point and one of the GM or WM barycenter point. The projection direction is the line that can be run between the barycenters of the two auxiliary materials, GW and WM.

Based on the so determined constellation of the clusters, that is the projection direction $\Pi$ and the base line B, density map determiner DMD determines the density map $\delta_{m0}$ for target material *m0*. Operation of target material density map determiner DMD may include a dimensional reducer DR that dimensionally reduces or "collapses" the geometric constellation of the clusters, using base line BL and projection direction $\Pi$. In embodiments, this dimensional reduction operation may include projecting along the projection direction $\Pi$, data points in A-space onto the base line for target material cluster. The whole or only a subset of spectral data as represented in the attenuation domain of A-space may be projected onto the base line BL so that each point in spatial domain corresponds to a point on the base line.

The points so projected will define a distribution which is bounded on the base line BL to the left and right along the line by two extremal points being at a maximum distance apart. Based on the maximum distance, a scalar coordinate of the projected points on baseline BL can be defined. This scalar coordinate may be defined as the distance of a given point from one of the extremal points, normalized for example by the maximum distance. The coordinates are scalars and as such are mappings of the projected points into a bounded interval, such as between 0 and 1. Other such mappings, preferably linear, may be used by density map determiner DMD to define coordinates for the projected data points on base line BL. The so defined coordinates along base line represents the proportion of the target material in a mixture comprising the target material and the combination of some or all auxiliary materials. Thus, contrast between the auxiliary materials themselves are eliminated, at the benefit of increased contrast of target material relative to the one or all of the auxiliary materials. For example, a scalar value coordinate "0" may be representative of 100% water in the mixture, whereas "1" may be representative of 0% water contribution.

The density map determiner DMD may use inverse transformer T$^{-1}$ to transform projected points on base line BL back into spatial domain, and assigning their respective base line scalar coordinate to the voxel position in spatial domain that corresponds to the said back-transformed point. In this manner, a scalar field can be defined by density map determiner DMD in 3D space. The scalar field is representative of the desired density map for the target material $\delta_{mo}$. The density map $\delta_{mo}$ may be stored in a memory, may be visualized by visualizer VIZ on display device DD, or may be otherwise processed as desired.

Whilst in embodiments it is the whole of the spectral data across the whole spatial domain that is mapped into A-space as described above, this is not necessarily so in all embodiments where processing of portions in spatial domain is also envisaged. In this embodiment a sub-set of the spatial domain is defined and processed as described above, with the processing confined to this subset. This allows focusing on specific portions of the anatomy of interest, with reduction of computational overhead and increased responsiveness. For example, the above-described computation of the water map (or for any other target material) can be done separately for different parts of the full brain volume. This separation may lower an impact of image artifacts, like shading or cupping, on the water map values. This because such artifacts may lead to a slight variation of the HU-values of the same tissues.

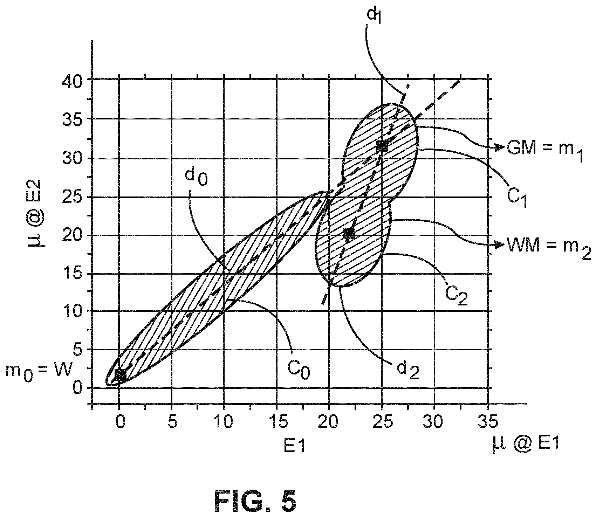

Reference is now made to Figure 5 which illustrates operation of the map density determiner system S-SYS for determining a water map in brain spectral imagery S. The A-space is defined by two monoenergetic images. In the initial point cloud formed by transformed data T(S) in A-space, three clusters c0, c1 and C2 are determined for three materials: water/CSF, grey matter GM and White matter WM, respectively. Respective directions *d0,d1,d2* are determined for the clusters c0,c1,c2. If there are, as shown, two separate clusters C1, C2 for the two auxiliary materials GM and WM, the respective directions d1, d2 can be averaged into a common direction and this is then used for the projection direction onto the water base line BL defined by direction d0. Alternatively, only one cluster is determined for GM or WM. Preferably, clustering is for GM.

The system S-SYS may be used for target material m0 with a single auxiliary material m1 mixture, but system performance may be more stable for mixtures with two or more auxiliary materials m1,m2. In a single auxiliary material, the proposed system may be used to reduce noise. As said, a cluster for the single or each of the two or more auxiliary materials is established each with its orientation to define the single or more projection directions $\Pi j$. If there are more than one projection directions, these maybe averaged or otherwise combined as mentioned above. Alternatively, separate projections are done for each auxiliary cluster and the projected values on the base line are then combined to obtain the density or concentration for the target material. If there a sufficient separation between the two auxiliary clusters, multiple such projection may be preferred, or their directions are averaged or otherwise combined to reveal a single projection. If there is no sufficient separation in A-space, the two or more auxiliary materials may be treated as a mixture in a common cluster with its direction the projection direction. The determiner DMD may include a separation power determiner logic configured to determine the level ("power") of separation. An information theoretic or statistical measure may be used for example with thresholding to decide how to process the auxiliary material clusters, as common cluster or separately as described.

Reference is now made to Figure 6 which shows a semi-automatic embodiment where the clustering is defined at least in part by user interaction provided through UI, such as GUI. The point cloud T(S) is visualized in graphics display on display device DD. The user may provide a segmentation of the point cloud into the clusters as shown by bounding boxes BB in heavy lines. A rough or coarse such segmentation may suffice. The bounding boxes BB enclose respective subset of points in the initial point cloud that the user, using their medical knowledge, estimates to define a respective cluster for water and GM,WM. The user may assign tags by UI to indicate which bounding boxes BB include the water (target material) cluster and which bounding boxes BB include GM and WM, respectively.

It may be sufficient to provide merely a single brain matter BM segmentation which represent the mix of GW and WM. Alternatively, separate cluster are defined, one for grey matter and one for white matter. Upon conclusion of the user input based segmentation, geometry component automatically determines base line BL and projection direction for each set of points in the respective bounding box. The water or CSF map may then be determined by map determiner DMD using projection onto base line BL and inverse transformation $T^{-1}$ into spatial domain as described above.

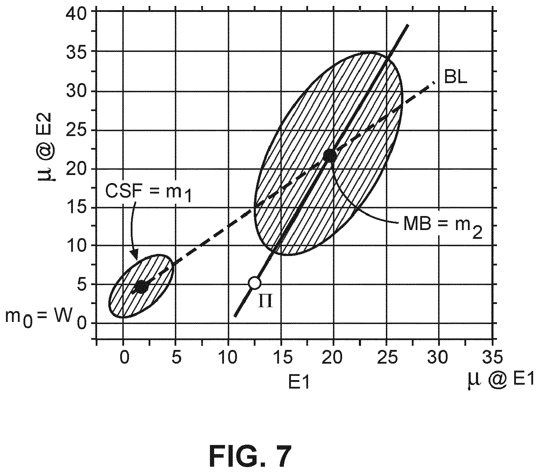

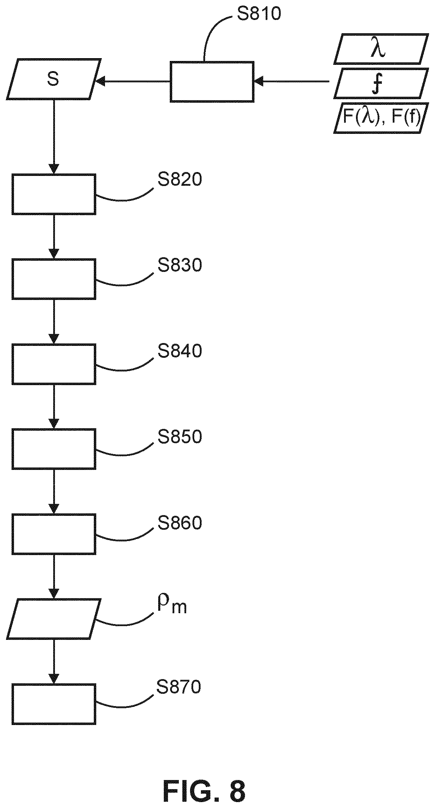

Reference is now made to Figure 7 which shows a different embodiment where the projection direction $\Pi$ is based on determining a maximal value or other designated value MB on the water or CSF base line BL. More details are provided below at Figure 8.

Reference is now made to Figure 8 which shows a flow chart of a computer-implemented method for generating density or concentration maps of target material, based on spectral data. The method may be understood as implementing the above-described system S-SYS, although this is not necessarily required herein and the described steps may also be understood as a teaching in their own right.

At step S810 spectral data S is generated. The spectral data S may be based on a spectral (multi-energy) projection data acquired by X-ray imaging apparatus configured for spectral imaging. Detector- or X-ray-source based spectral acquisition hardware may be used. The spectral data S may include data computationally derived from spectral projection data by a spectral data processing algorithm F. The spectral data may include spectral image data $f$ reconstructed in image domain based on the spectral projection data image data by a spectral tomographic reconstruction algorithm. The reconstructed spectral data may represent a 3D volume or cross-sectional imagery of the ROI. The spectral projection data may be processed first by a spectral data processing algorithm $F$, and it is the so processed data that is then reconstructed. This may be referred to as projection-based methods. Alternatively, the spectral projection data is reconstructed first, and the reconstructed spectral image data is then processed by the spectral data processing algorithm F. This may be referred to herein as image-based methods. The spectral data processing algorithm F may include spectral separation algorithms. In spectral separation algorithms, the spectral projection data is used to derive spectral data that allows separating the earlier mentioned manners of matter-versus X-ray interaction. For example, attenuation cause by one base material may be separated from attenuation caused by another base material. Another example includes separating attenuation cause by Compton scattering versus attenuation caused by photoelectric absorption. Thus, there exist projection-based material/Compton vs Photoelectric separation and image-based material/Compton vs Photoelectric separation, both envisaged herein in embodiments.

The separation may be based on solving as system of linear equations ( see eq (1) below) in as many unknowns as there are energy levels Ej. This was observed by R E Alvarez & A Macovski et al, published in "Energy-selective reconstructions in X-ray computerized tomography", Phys Med Biol, Vol 21(5): 733-44 (1976). However, because of better noise management, the proposed target material density map generation is preferably based on projection-based material/Compton vs Photoelectric separation.

Preferably, generating S810 the spectral data includes computing base images with a projection-based separation algorithm and, based on the base images, two mono-energetic images. For example, in embodiments, step S810 includes applying projection-based separation to compute two base images ("base function"). For example, bone and water (or any other material pair with sufficiently different attenuation behavior) may be used as base functions/materials to obtain a bone base image and a water base image. Note, this water image is different from the water map that be computed herein, on which more further below. A separation in Compton and Photelectric image may be used instead to obtain the base images. An optional denoising may be used to reduce in particular anti-correlated noise in the base images. Using the base images, at least two different virtual mono-energetic images for E1, E2 are computed. For example, 40 keV and 70 keV, or 50 keV and 75 keV may be used as energy values E1, E2. Thus, the spectral data S may include the (at least) two mono-energetic images. Alternatively, the spectral data S includes only the base images (photo-Compton, or bone-water) without prior calculation of virtual mono-energetic images.

Step S810 may include transforming via transformation $T$ the spectral data S from spatial domain into A-space.

Spectral data thus may be represented as points in an at least two-dimensional attenuation space, with attenuation values at different energies related to each other as described above. However, other A-space representations are also envisaged, herein, depending om the type of spectral data and the spectral data processing algorithm F used. This transformation is preferably reversable in the general sense, so that each data point $p$ in A-space can be assigned back to one or more voxels in spatial domain: $T^{-1}$ ( $p$=($HU1$, $HU2$) $\in$ A-space) = {all voxels x | ray though voxel x experienced attenuation $HU1$ and $HU2$ at energies $E1$, $E2$}.

At step S820 the preferably transformed spectral data is received.

At step S830 clusters for target material and at least one other auxiliary material is determined. At least one cluster is determined for the target material and at least one other cluster is determined for an auxiliary material or for a mix of such auxiliary materials.

Step S830 may include a filter step to reduce the number of data points in A-space to be processed in the following steps. This step may be based on prior anatomical knowledge. For example, portions of the ROI where the target material occurs naturally (in the non-pathological case) may be excluded. The filter step may be applied in spatial domain before transformation T, or may be done in A-space. For example, the filter step may include thresholding an applicable range of the value distributions of the combined GM/WM voxel distribution, to so exclude from processing rogue values caused by artifacts, materials of no interest, etc. Such identification may be implemented by thresholding in a kernel density map or by an interactive segmentation in A-space based on user input. Alternatively, GM/WM (= brain matter) voxels and/or CSF voxels are identified by using automatic segmentation in bone/brain/CSF, which can be done by various otherwise known methods, such as watershed segmentation, machine learning models, or any other approach.

Optionally, partial volume voxels of water/CSF and GM are identified in the filter operation in step S830. For example, to find CSF-GM base line, a transition area in A-space between CSF and GM may include mainly partial voxels representing a mixture of CSF+ GM. This area may be identified by user other automatically using PCA or other.

For step S830, any clustering method in A-space can be used in the filtered or non-filtered point cloud, such as PCA, machine learning based (eg, $k$-means clustering), EM(expectation-maximization)-algorithm, statistical mixture models, and others.

The machine learning model may be trained using any machine learning algorithm such as back-propagation or other gradient based techniques. Historic spectral data may be used, suitable annotated by a human expert. The annotations may indicate location and orientation of clusters. Neural network type models may be used, in particular of the convolutional type.

At step S840 the geometrical constellation between the clusters is determined. This step may include determining a respective direction for some or each cluster in A-space. The direction of the target cluster is the base line BL, and the direction for the auxiliary material(s) cluster is the projection direction $\Pi$. The directions may be computed by fitting least square lines, by PCA or other. Alternatively, lines between barycenters of the clusters are cast: one from the barycenter of the target cluster to the barycenter of one of the auxiliary material clusters to obtain baseline BL. Projection direction may be defined by line run between barycenters of the two auxiliary material clusters.

In yet another embodiment (which may be combined with any of the above), step S840 may include receiving an automatic or user facilitated segmentation in A-space to define the target material and the auxiliary material(s). A coarse segmentation by way of bounding boxes may be sufficient as described above in Figure 6. For example, water and GM may be so segmented. The data points $p$ in the, for example, water and GM segments, may be averaged , for example component wise for HU1 and HU2, to determine the base line BL as the direction of the corresponding line between the average water- and GM-values in A-space. This segmentation with subsequent averaging in the segments may be applied to any target and auxiliary material.

As an alternative, the direction of one of the clusters is determined based on a designated (single) value along the direction of the other cluster. For example, as shown in Figure 7, the designated value may be an average or maximum value. For example, the maximum value ($\approx$ mean brain, MB) on the major axis of the combined GM and WM distribution is determined. The major axis of the GM+WM cluster is the projection direction. The base line may then be determined as the line that runs between the water or CSF cluster and the designated point MB. The point in A-space for this line BL may be defined from the respective clusters for example as barycenters, or other well-defined points. Determining the designated point MB along the direction of the auxiliary material cluster may be done more accurately in some instances, as compared to computing the major axis of the target material cluster using PCA or other.

At step S850, based on the determined geometric constellation defined by the directions of the clusters, the density map is determined.

Step S850 may include a dimensionally reducing A-space using the geometric constellation of the clusters. In one embodiment, A-space points are projected onto the base line of the cluster defined for the target material along the projection direction determined by direction of the one or more auxiliary material cluster(s). Thus, in embodiments, the geometrical constellation is collapsed onto a sub-space defined by the data points that form the target material cluster.

The dimensional reduction operation at S850 may include the whole of the data space or may include only parts thereof.

Co-ordinates in the sub-space (such as the base line) on which the points in attenuation space are projected onto are determined. The set of points projected onto the base line may be bounded by two extremal points on the base line. In the 2D case, the coordinates may be determined as a scalar, such as a distance of the projected points along the base line from one of the extremal points. Each projected point in A-space has thus a such a scalar coordinate. Different points may have the same coordinates on the base line. The so determined scalar coordinate for a given projected point is then a measure for the density or concentration of the target material in the imaged ROI. Step S850 may include inversely mapping back points in A-space into spatial domain and assign to each such point in spatial domain its respective scalar value (the above-mentioned coordinate in the subspace formed by the base line BL).

At step S860, the so determined scalar values spatially assigned to locations in spatial domain are made available as the density map $\delta_{m0}$ for the target material.

At step S870 the so density map $\delta_{m0}$ may be visualized, stored or further processed or analyzed, as required.

For example, an analyzing step S870 may include using the map in a diagnostic algorithm to diagnose for stroke. For example, the step S870 may include comparing the water density map against threshold values to conclude whether a stroke or edema is present in a given patient. The above-described method may be preferably used for brain imaging but other applications for other organs, organ types or groups of organs, or even outside the medial field, are also envisaged herein where a quantitative determination of densities of one or more target materials is called for.

It will be understood that the above-described principles admit to various generalizations, all envisaged herein in different embodiments.

Specifically, the representation of A-space in two dimensions for attenuation at two energy windows *E1*, *E2* as illustrated above may be extended to more than two dimensions. This extension to three or more dimensions may be considered for spectral data acquired by imagers IA with acquisition circuitry capable of resolving for more than two energy windows, such as detectors with photon-counting circuity, detectors with more than two layers, multi-source imagery, etc. The above-described principles are all applicable, expect that the "base line" is now defined as a subspace of more than 1 dimension, such as an affine subspace BL, with dimension *N-1,* N<2 being the dimension of the extended A-space. For example, for *N*= 3, the subspace BL for the target material is a plane. The projected coordinates in such a more than 1D subspace BL are also more than 1D and may need to be combined by functional into a scalar representative of the density of the target material. For example, the squared sum functional $\sum_i x_i^2$ or other may be used.

In addition to the extension into more than 2D, the representations of the A-space in terms of other than attenuation values at different energies are envisaged herein, depending on the spectral data processing used. For example, the dimensions of A-space may be spanned instead by contributions of attenuation from photoelectric absorption versus Compton scattering. In yet a different embodiment, the A-space may be spanned by the respective attenuation suffered from different material types. Such material specific attenuation contributions may be obtained by material decomposition algorithms as implemented by the spectral data processor SDP. The material decomposition may be done in projection domain or image domain. Specifically, the caid coefficients are obtained as follows. Any material with an attenuation coefficient $\mu_m(E)$, it can be well approximated by a linear combination of two "base functions", for instance in the form:

$$\mu_m(E) = a_{m1} \cdot \mu_{m1}(E) + a_{m2} \cdot \mu_{m2}(E) \qquad (1)$$

According to Alvarez et al, using two energy levels E1, E2 allows solving (1) for the two coefficients $a_{m1}$, $a_{m2}$. Thus, the A-space coordinates may be spanned by the $\mu_{m1}(E)$ and $\mu_{m2}(E)$ respectively.

By way of a further observation, in material decomposition techniques based on (1) or similar, imaging with spectral data at two energy levels *E1*, *E2* allows solving (1) for coefficients $a_{m1}$, $a_{m2}$ to obtain material images, such as a bone and water image in a (m1 = bone, m2= water) decomposition. The "water image" image may be obtained by displaying the coefficient $a_{m1=water}$ in 3D-space for example. However, such as "water image" is markedly different from the proposed water or other target material map. The proposed target material map as computed herein is less artifact-prone, more quantitative and more accurate as a mere material image from a spectral material decomposition as per (1). The proposed map is not based on coefficients (1), but instead on the explained projection on the base line/subspace BL as defined above. Specifically, a difference between such as target material image from a material decomposition and the target material map as computed herein in that the latter (such as the water map) better represents relative deviations from a normal or expected concentration of the target material, rather than absolute concentrations as captured in target material image. For the example, in the case of water as target material, the water map represents deviations of water concentrations from the "normal" or expected water concentrations in healthy WM or GM. In the water image on the other hand, there is contrast for absolute water concentration. This may cause for present purposes irrelevant contrast between healthy GM/WM according to their natural water content. Thus, the proposed water map may be better suited than the water image for applications where an accurate deviation of "healthy" water content is called for, such as for stroke diagnosis as envisaged herein in embodiments".

It will be appreciated that the above system and methods harnesses the observation that the material clusters definable in A-space admit a definition of directionality as observed above, for example in terms of major axis as distinguished from a width, or minor axis in a PCA setup. It is believed that this phenomenon at least partly derives from a certain "asymmetric" noise behavior. Specifically, a width and orientation of the HU value distribution of material clusters is already determined by the noise in the base images (e.g. bone-water or photo-Compton images) after optional denoising. A reason for the characteristic noise distribution that allows definition of such directionality can be derived from the different energy dependency of the attenuation base functions: for example, in water-like brain tissue the base material coefficients have a very different dependence on energy. And the photoelectric coefficient has a much larger variation on energy than the Compton scatter coefficient. Thus, in case of a bone-water base material choice for spectral data S generation, the bone coefficient ( see eq (1) ) has a much larger variation than the water coefficient. Therefore, the noise along the photoelectric axis is much larger than along the Compton-scatter axis. The same holds for the 'bone' direction with respect to the 'water' direction in case of a bone-water basis. The long axis of the noise distribution is therefore nearly parallel to the photoelectric or bone axis, which causes a predetermined long axis of the noise distribution in A-space for the two different virtual mono-energetic images. As the difference in elemental composition between GM and WM is mainly the lipid content as observed earlier, this leads in a photo-Compton separation to a pronounced difference in the photoelectric coefficient and in a water-bone separation to a dominant difference in the bone coefficient, respectively. Therefore, the major axis of the noise distribution essentially coincides with the line between GM and WM, and can therefore be used as projection direction for the proposed water map calculation. This effect also reduces noise in the water map itself derived by projection onto the base line. Whilst there may be noise in projection direction itself, does not feed through into the water map itself. The major axis of the HU values distribution of GM and WM is therefore indicative of the projection direction for the proposed water map calculation. As mentioned, the projection direction can be either determined separately for the GM and WM clusters, of, if there is too much of an overlap between the two, for the combined distribution in a single step. Whilst the above is explained mainly with reference to a PCA setup ( minor/major axes of ellipses/ellipsoids), this is merely an example and the above explained directionality engendered by the asymmetric noise behavior may be harnessed for present purposes equally in the context of other clustering techniques, such as least squares of others still.

The components of system S-SYS may be implemented as one or more software modules, run on one or more general-purpose processing units PU such as a workstation associated with the imager IA, or on a server computer associated with a group of imagers. The system S-SYS or components thereof may be integrated into the imager IA.

Alternatively, some or all components of system S-SYswitch S may be arranged in
hardware such as a suitably programmed microcontroller or microprocessor, such an FPGA (field-programmable-gate-array) or as a hardwired IC chip, an application specific integrated circuitry (ASIC), integrated into the imaging system SIS. In a further embodiment still, the system S-SYS may be implemented in both, partly in software and partly in hardware.

The different components of system S-SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It should be noted that embodiments of the invention are described with reference to different subject matters. In particular some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

**Claims**

1. A system (S-SYS) for determining a material density map for a target material, configured to:

   receive at least spectral data representable in an at least two-dimensional data space, the spectral data including, or based on, measurements acquired by a spectral imaging apparatus of at least a part of an object in a three-dimensional image domain of the spectral imaging apparatus;
   determine clusters in the data space, one indicative of the target material, the target material cluster, and at least one other cluster indicative of at least one auxiliary material, the auxiliary material cluster(s);
   determine a mutual geometrical constellation of the clusters; and
   determine the said material density map based on the geometrical constellation so determined.

2. The system of claim 1, wherein the system is to visualize said material density map in the said imaging domain on a display device.

3. System of claim 1 or 2, wherein the determining of the mutual geometrical constellation of the clusters by the system includes determining at least one respective orientation of at least one of the clusters relative to the at least one other cluster.

4. System of any one of the previous claims, wherein the determining of the said material density map by the system includes dimensionally reducing the said spectral data.

5. System of claim 4, wherein the determining of the said material density map by the system includes projecting the auxiliary material cluster on a subspace defined by the target material cluster.

6. System of any one of the previous claims, wherein the clusters and/or at least one orientation is determined by any one or more of: i) principal component analysis, PCA, ii) a trained machine learning model, iii) segmentation operation.

7. System of any one of the previous claims, wherein the system includes or is coupled to a user interface (UI) configured to allow a user to define the said clusters and/or their orientation based on visual representation of the spectral data on the, or a, display device, wherein the user interface is preferably a graphical user interface.

8. System of any one of the previous claims, wherein the spectral imaging apparatus is any one of: i) an X-ray imager, ii) a computed tomography, CT, scanner iii) or tomosynthesis scanner.

9. System of any one of claims 3-8, wherein the determining of the orientation of the target material cluster is based on at least one value in the auxiliary material cluster.

10. System of any one of the previous claims, wherein the material density map is determined separately for different parts of the image domain.

11. System of any one of the previous claims, wherein the at least one auxiliary material includes one or more of grey matter and white matter, and/or wherein the target material includes water or cerebrospinal fluid.

12. Imaging arrangement (SIS) comprising a system (S-SYS) any one of the previous claims, and the spectral imaging apparatus (IA).

13. Computer-implemented method for determining a material density map for a target material, comprising:

receiving (S820) at least spectral data representable in an at least two-dimensional data space, the spectral data including, or based on, measurements acquired by a spectral imaging apparatus of at least a part of an object in a three-dimensional image domain of the spectral imaging apparatus;
determine (S830) clusters in the data space, one indicative of the target material, the target material cluster, and at least one other cluster indicative of at least one auxiliary material, the auxiliary material cluster(s);
determine (S840) a mutual geometrical constellation of the clusters; and

determine (S850) the said material density map based on the geometrical constellation so determined.

14. A computer program element, which, when being executed by at least one processing unit (PU), is adapted to cause the processing unit (PU) to perform the method as per claim 13.

15. At least one computer readable medium having stored thereon the program element of claim 14, or having stored thereon the machine learning model as per claim 6.

**FIG. 1**

**FIG. 1A**

FIG. 2

FIG. 3

**FIG. 4**

FIG. 5

FIG. 6

FIG. 7

**FIG. 8**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NARUTO NORIHITO ET AL: "Dual energy computed tomography for the head", JAPANESE JOURNAL OF RADIOLOGY, SPRINGER JAPAN, TOKYO, vol. 36, no. 2, 9 November 2017 (2017-11-09), pages 69-80, XP036422555, ISSN: 1867-1071, DOI: 10.1007/S11604-017-0701-4 [retrieved on 2017-11-09] * abstract; figures 2,9-12 * * sections: 2MD Algorithm; 3MD Algorithm, X-Map algorithm. * | 1-15 | INV. G06T7/00 |
| X | PETRONGOLO MICHAEL ET AL: "Noise Suppression for Dual-Energy CT Through Entropy Minimization", IEEE TRANSACTIONS ON MEDICAL IMAGING, IEEE, USA, vol. 34, no. 11, 1 November 2015 (2015-11-01), pages 2286-2297, XP011588709, ISSN: 0278-0062, DOI: 10.1109/TMI.2015.2429000 [retrieved on 2015-10-30] * abstract; figure 1 * | 1,3-15 | |
| | | | TECHNICAL FIELDS SEARCHED (IPC) G06T A61B |
| A | EP 3 555 860 A1 (KONINKLIJKE PHILIPS NV [NL]) 23 October 2019 (2019-10-23) * abstract; figure 9 * * paragraphs [0007], [0033], [0034] * | 1-15 | |

The present search report has been drawn up for all claims

| Place of search | Date of completion of the search | Examiner |
|---|---|---|
| Munich | 10 May 2022 | Rimassa, Simone |

EPO FORM 1503 03.82 (P04C01)

## ANNEX TO THE EUROPEAN SEARCH REPORT
## ON EUROPEAN PATENT APPLICATION NO.

EP 21 21 4586

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2022

| Patent document cited in search report | | Publication date | Patent family member(s) | | Publication date |
|---|---|---|---|---|---|
| EP 3555860 | A1 | 23-10-2019 | CN | 110574073 A | 13-12-2019 |
| | | | EP | 3555860 A1 | 23-10-2019 |
| | | | US | 2020027253 A1 | 23-01-2020 |
| | | | WO | 2018114964 A1 | 28-06-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **K NOGUCHI et al.** A Novel Imaging Technique (X-Map) to Identify Acute Ischemic Lesions Using Noncontrast Dual-Energy Computed Tomography. *J Stroke Cerebrovasc Dis,* 2017, vol. 26 (1), 34-41

- **R E ALVAREZ ; A MACOVSKI et al.** Energy-selective reconstructions in X-ray computerized tomography. *Phys Med Biol,* 1976, vol. 21 (5), 733-44